# EUROPEAN PATENT APPLICATION

(11) **EP 1 839 485 A1**
(43) Date of publication of application: **03.10.2007**
(21) Application number: 06006608.1
(22) Date of filing: 29.03.2006
(51) Int. Cl.: A01K 67/027, C07K 14/47, C12N 15/10

(54) **Animal model based on targeted apoptosis for the study of genetic diseases such as Parkinson's disease**

(71) Applicant: Deutsches Krebsforschungszentrum Stiftung des öffentlichen Rechts, 69120 Heidelberg (DE)
(72) Inventor: Grummt, Ingrid, 69118 Heidelberg (DE); Parlato, Rosanna, 64625 Bensheim (DE); Schütz, Günther, 69120 Heidelberg (DE); Xuejun, Yuan, 69214 Eppelheim (DE); Kreiner, Grzegorz, 69120 Heidelberg (DE); Rieker, Claus, 69115 Heidelberg (DE)
(74) Representative: Schüssler, Andrea

(57) **Abstract**

Described is the use of a non-human transgenic animal, preferably a mouse, characterized in that it contains a modified version of the gene encoding TIF-IA (a) as an animal model for a disease, (b) for analyzing the function of selected stem cells or (c) for ablating malignant cells or microglia cells. Furthermore, methods for screening a therapeutic compound are described which comprise administering a candidate compound to said non-human transgenic animal (or a cell line derived from said non-human transgenic animal) and monitoring a therapeutic effect of said compound.

SEQUENCE LISTING

<110> Deutsches Krebsforschungszentrum Stiftung des öffentlichen
      Rechts

<120> Animal model based on targeted apoptosis for the study of genetic
       diseases such as Parkinson´s disease

<130> K 2343EP

<140> EP06006608
<141> 2006-03-26

<160> 4

<170> PatentIn version 3.2

<210> 1
<211> 30
<212> DNA
<213> artificial

<220>
<223> Primer

<400> 1
ccggtggtcc tgcttacact agagatgtgg                                    30


<210> 2
<211> 32
<212> DNA
<213> artificial

<220>
<223> Primer

<400> 2
aatataattt gcagcagcct gcctgatgat gg                                 32


<210> 3
<211> 20
<212> DNA
<213> artificial

<220>
<223> Primer

<400> 3
ctgccaggga catggccagg                                               20


<210> 4
<211> 21
<212> DNA
<213> artificial

<220>
<223> Primer

<400> 4
gcacagtcga ggctgatcag c                                             21

## Description

The present invention relates to the use of a non-human transgenic animal, preferably a mouse, characterized in that it contains a modified version of the gene encoding TIF-IA (a) as an animal model for a disease, (b) for analyzing the function of selected stem cells or (c) for ablating malignant cells or microglia cells. The present invention also relates to methods for screening a therapeutic compound which comprise administering a candidate compound to said non-human transgenic animal (or a cell line derived from said non-human transgenic animal) and monitoring a therapeutic effect of said compound.

Parkinson's disease is one of a larger group of neurological conditions called motor system disorders. It was first described as "the shaking palsy" in 1817 by James Parkinson. Because of Parkinson's early work in identifying symptoms, the disease came to bear his name. In the normal brain, some nerve cells produce dopamine, which transmits signals within the brain to produce smooth movement of muscles. In Parkinson's patients, 80 percent or more of these dopamine-producing cells are damaged, dead, or otherwise degenerated. This causes the nerve cells to fire wildly, leaving patients unable to control their movements. Symptoms usually show up in one or more of four ways:
- tremor, or trembling in hands, arms, legs, jaw, and face
- rigidity, or stiffness of limbs and trunk
- bradykinesia, or slowness of movement
- postural instability or impaired balance and coordination.

Several structures of the brain are related to Parkinson's disease. Basal ganglia affect normal movement and walking; neurons in the substantia nigra produce the neurotransmitter dopamine and thereby control muscle movements. The globus pallidus is part of a larger structure connected to the substantia nigra affecting movement, balance and walking.

So far, the usual treatment of Parkinson's disease is a combination of levodopa and carbidopa. Levodopa, which affects neurochemical abnormality, revolutionized treatment. However, over the years, its effectiveness can decline and its side effects, such as motor complications, can increase. Adjusted dosage can help but additional medications may be required. Because of levodopa's complexities, young people with Parkinson's often start with other treatments, reserving levodopa for later in the disease. Patients have other treatment options, including surgery. Transcranial magnetic stimulation is also being studied. So far, the experimental studies used for the detection/evaluation of new candidate drugs which might be useful for the treatment of Parkinson's disease were mainly based on a model system using the neurotoxin MPTP (1-methyl-4-phenyl-1,2,3,6-tetrahydropyridine) which, unfortunately, has severe disadvantageous effects, i.e., generates severe and irreversible symptoms in humans, primates and other mammals. An important disadvantage of the MPTP-induced model is that the changes occur rapidly and do not show the chronic development as characteristic of the Parkinson's disease.

Thus, the technical problem underlying the present invention is to provide a new animal model allowing to study neurological diseases like Parkinson's disease which overcomes the disadvantages of the presently used models.

The solution to said technical problem is achieved by providing the embodiments characterized in the claims. During the experiments leading to the present invention it was found that the solution to the above defined technical problem can be achieved by interfering with the function of the transcription factor TIF-IA allowing to regulate the survival of selected target cells. TIF-IA is the key player in growth-dependent control of rDNA transcription and is the mammalian homologue of yeast Rrn3p. TIF-IA was initially identified as an activity that complements transcriptionally inactive extracts obtained from quiescent mouse cells. TIF-IA activity is for example reduced in stationary phase cells, after drug-induced inhibition of protein synthesis and deprivation of essential nutrients. The molecular mechanisms that modulate TIF-IA activity in response to cell growth are unknown. TIF-IA has been shown to interact with both Pol I and two TAFs of the TBP-containing factor TIF-IB/SL1.

Accordingly, the function of a specific cell or tissue of an animal can be inhibited in the animal model of the present invention which is based on cell/tissue specific inactivation of the ubiquitously expressed TIF-IA. This model allows to simulate a variety of neurological disorders, e.g., Parkinson's disease and to establish new therapeutic approaches. In the mouse model of the present invention it was found that inactivation of the TIF-IA encoding gene within the germ line results in early lethality at day 8.5. Loss of the TIF-IA gene in embryonic fibroblasts leads to apoptosis. These observations were the starting point for the present invention: Using the Cre/loxP system which allows to generate cell specific and regulated inactivation of a gene, the function of the TIF-IA encoding gene was turned off in selected somatic cells. Importantly, TIF-IA inactivation in dopaminergic neurons by expression of the recombinase exclusively in these neurons allowed to develop a novel model for Parkinson's disease. Thus, by eliminating the function of TIF-IA in dopaminergic neurons, which subsequently lead to loss of dopaminergic neurons preferentially in the substantia nigra, an animal model for Parkinson's disease can be established allowing, e.g., to screen for new drugs for therapy. Moreover, by inactivating the TIF-IA gene in other tissues the animal model of the present invention is useful for the study of further diseases, e.g.,
(a) Huntington's chorea by inactivating TIF-IA in striatal neurons;
(b) amyotrophic lateral sclerosis by inactivating TIF-IA in motorneurons;
(c) sleep-wakefulness by targeted destruction of noradrenergic and/or histidinergic and/or serotonergic neurons;
(d) spinal or bulbar muscular dystrophia by ablating neurons expressing androgen receptors;
(e) diabetes by targeted destruction of ß-cells of the pancreas; and
(f) endocrinic diseases by targeted destruction of the cells responsible for these diseases.

Moreover, the animal model of the present invention is useful for
(a) ablating microglia cells by expressing Cre recombinase under the control of the lysozyme gene promoter;
(b) analyzing the function of selected stem cells (e.g., neural stem cells) by targeted ablation in vivo; and
(c) targeted ablation of malignant cells by cell specific inactivation of TIF-IA using RNAi respectively.

### Brief description of the drawings

Figure 1:
   (A) Schematic re-presentation of the region of exons 11 to 15 of the TIF-IA encoding gene
      Black triangles symbolize loxP sites flanking exons 12 to 14.
   (B) Schematic re-presentation of the region of exons 11 to 15 of the TIF-IA encoding gene
      Exons 12 to 14 have been excised by the iCre recombinase (one loxP site is maintained).
Figure 2: Massive p53 upregulation and activation of caspase-3 in the ventricular zone of TIF-IA NesCre mutants at E12.5. Sagittal paraffin sections at E12.5 from control (A, C) and TIF-IA^{NesCre} mutant (B, D). Sections immunostained with a p53-specific antibody showing upregulation of p53 in the ventricular zone (VZ) of the TIF-1A^{NesCre} mutant (B). Adjacent sections immunostained with anti-activated caspase-3 antibody show high levels of apoptosis in the VZ of the TIF-1A^{NesCre} mutant (D).
Figure 3: Schematic re-presentation of the DATCre transgene Transcription of the gene encoding iCre recombinase is controlled by the dopamine transporter promoter (DAT promoter).
Figure 4: Cre recombinase is expressed exclusively in neurons expressing the dopamine transporter (DAT) gene.
   Sagittal vibratome sections of brains of 2 months old animals carrying the DATCre transgene. (A) Dopaminergic neurons in SN/VTA in sagittal sections are visualized by DAT mRNA in situ hybridization (blue) in the ventral mesencephalon. (B) High magnification of the region containing the SNpc in 2 months old DATCre expressing mice. Dopaminergic neurons are identified by non-radioactive in situ hybridisation using a specific riboprobe for DAT mRNA (blue staining), in combination with immunohistochemical detection of the Cre recombinase (brown staining). The double staining shows that dopaminergic neurons expressing DAT mRNA also express the Cre recombinase.
Figure 5: Fig. 5: Selective loss of tyrosine hydroxylase immunoreactivity in substantia nigra at the age of 1 month.
   Coronal sections of 1 month old TIF-IA^{DATCre} mice, stained with a tyrosine hydroxylase-specific antibody. Loss of tyrosine hydroxylase immunoreactivity in the substantia nigra pars compacta (SNpc) is clearly visible, but not in the ventral tegmental area (VTA). (A) control mouse; (B) mutant mouse.
Figure 6: Ablation of TIF-IA leads to p53 upregulation at P7. Coronal vibratome sections of TIF-IA ^{DATCre} mutant and control mice processed with different antibodies: (A, B) Tyrosine hydroxylase immunoreactivity in substantia nigra on coronal sections at P7. (C, D) Adjacent sections analyzed by a Cre-specific antibody. (E, F) p53 protein visualized by immunohistochemical detection in substantia nigra.
Figure 7: Progressive loss of tyrosine hydroxylase immunoreactivity in the striatum.
   Coronal sections from controls and TIF-IA^{DATcre} mutants at different postnatal stages (P7, P30) stained with a tyrosine hydroxylase-specific antibody in the striatal region. (A, B) Control and mutant at P7, (C, D) Control and mutant at P30.

Thus, the present invention relates to the use of a non-human transgenic animal characterized in that it contains a modified version of the gene encoding TIF-IA (a) as an animal model for a disease, (b) for analyzing the function of selected stem cells or (c) for ablating malignant cells or microglia cells,wherein said modified version of the gene encoding TIF-IA is a version that is cell-specifically or tissue-specifically modified.

By means of conventional molecular biological processes (see, e.g., Sambrook et al., Molecular Cloning, A Laboratory Manual 2nd edition (1989) Cold Spring Harbor Laboratory Press, Cold Spring Harbor, NY.) the modification of the TIF-IA encoding gene (Bodem et al., 2000; Schnapp et al., 1990; Yamamoto et al., 1996) can be introduced. For the manipulation in prokaryotic cells by means of genetic engineering the TIF-IA encoding nucleic acid molecules can be introduced into plasmids allowing a modification of a sequence by recombination of DNA sequences. By means of conventional methods (cf. Sambrook et al., supra) bases can be exchanged and natural or synthetic sequences can be added. In order to link the DNA fragments with each other adapters or linkers can be added to the fragments.

Furthermore, manipulations can be performed that provide suitable cleavage sites or that remove superfluous DNA or cleavage sites. If insertions, deletions or substitutions are possible, in vitro mutagenesis, primer repair, restriction or ligation can be performed. As analysis methods usually sequence analysis, restriction analysis and other biochemical or molecular biological methods are used.

In a preferred use of the non-human transgenic animal the modified version of the TIF-IA - encoding gene is a version that had been inactivated, preferably by deletion.

Particularly preferred are deletions of one or more exons with deletion of exons 12 to 14 being particularly preferred. The modification of the TIF-IA encoding gene of the non-human transgenic animal can be heterozygous or homozygous. The animal model is preferably from a genus of Mus (e.g. mice), Rattus (e.g. rats), Oryctologus (e.g. rabbits) and Mesocricetus (e.g. hamsters). More preferably the animal is a mouse or rat. The specific modification can be performed by homologous recombination, preferably in mouse embryonic stem cells by Cre/Lox-mediated homologous recombination (Hardouin and Nagy, 2000).

This novel transgenic mouse model is useful to assay for molecules and test experimental therapies. Furthermore, transgenic mice offer the additional advantage for large scale-testing of candidate drugs because of their small size and fast breeding propensities. Said mouse model is also likely to yield novel insights in the molecular mechanisms of neurological diseases, e.g., Parkinson's disease. Moreover, cell lines can be derived from the transgenic animal model. Derived cell lines can be heterozygous or homozygous for the above described modification of the TIF-IA gene. Typical but not limited examples of cell lines are fibroblasts and keratinocytes. Since it might be that homozygous mice are lethal at a particular stage of embryonic development it might not be possible to obtain particular homozygous mutant cells from transgenic animals. Thus, it is possible to start from homozygous mutant embryonic stem cells (ES) which can be efficiently differentiated in vitro. Said ES cells can be isolated from homozygous transgenic animals. It is well known in the art that cells from the inner cell mass of mammalian blastocysts can be maintained in tissue culture under conditions where they can be propagated indefinitely as pluripotent embryonic stem (ES) cells (Thomson et al., 1998). As such blastocysts from normal and double homozygous mutant mice can be rescued from 2 days old embryos. Alternatively, normal and homozygous mutant ES cells can be generated by high G148 selection of the heterozygous mutant ES cells. These stem cells can then be induced to differentiate into pure cultures using the genetic selection procedure described by Maltsev et al. (1994, Circ Res 75., 233-44).

Cell lines described above can be used for the development of a method for the screening of candidate compounds and monitoring the effectiveness of said compounds. It is expected that homozygous mutant cell lines are a better model than heterozygous mutant cell lines. Alternatively cell lines transfected with the human TIF-IA gene offer an alternative model for screening. Suitable cell lines can also be transfected with this mutant gene and used for screening purposes. Other cell systems, often used in electrophysiological studies, are oocytes of Xenopus laevis. In oocytes the mutated TIF-IA gene can for example be introduced by microinjection.

Preferred uses of the non-human transgenic animal as an animal model for a disease relate to the following diseases: Parkinson's disease, Huntington's chorea, amyotrophic lateral sclerosis, sleep-wakefulness, spinal or bulbar muscular dystrophy, diabetes and endocrine diseases. The present invention also relates to screening methods which are based on the above described non-human transgenic animal or cells derived therefrom.

Thus, the present invention also relates to a method for screening a therapeutic compound for use in the treatment of a disease as described above comprising:
(a) administering a candidate compound to the non-human transgenic animal; and
(b) monitoring a therapeutic effect of said compound.

In an alternative embodiment, the present invention relates to a method for screening a therapeutic compound for use in the treatment of a disease as described above comprising:
(a) administering a candidate compound to a cell line derived from the non-human transgenic animal; and
(b) monitoring a therapeutic effect of said compound.

The person skilled in the art can establish such screening assays according to well known methods and based on the known pathological effects on metabolism associated with such diseases.

Suitable candidate molecules for screening may be small molecules, biological polymers, such as polypeptides, polysaccharides, polynucleotides, and the like. Small molecules, e.g. small organic molecules, and other drug candidates can be obtained, for example, from combinatorial and natural product libraries. The test compounds will typically be administered to the culture-medium at a concentration in the range from about 1 nM to 1 mM, usually from about 10 µM to 1 mM. Monitoring can be measured using standard biochemical and electrophysiological techniques. These assays may be performed using conventional techniques developed for these purposes in the course of screening. To perform drug screening assays, it is feasible to accommodate automation of the assay. Interaction (e.g., binding of) between the recombinant cells and the target molecules can be accomplished in any vessel suitable for containing the reactants. Examples of such vessels include microtiter plates, test tubes, and microcentrifuge tubes.
The above methods are also useful for the evaluation of possible side effects of new or existing medicaments. Usually such side effects are not easily detected in a clinical study before a new medicine enters the market.

The molecules obtained in the above described screening methods can be used for the preparation of a medicament. The term 'medicament for treatment' relates to a composition comprising molecules as described above and a pharmaceutically acceptable carrier or excipient (both terms can be used interchangeably). Examples of suitable pharmaceutical carriers or excipients are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Administration of the medicament may be effected by different ways, e.g. by intravenous, intraperitoneal, subcutaneous, intramuscular, topical or intradermal administration. The route of administration, of course, depends on the nature of the disease and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind and stage of the condition or disease, general health and other drugs being administered concurrently.

Generally, the medicament is administered so that the compound obtained by the above described screening methods is given at a dose between 1 µg/kg and 10 mg/kg, more preferably between 10 µg/kg and 5 mg/kg. Preferably, it is given as a bolus dose. Continuous infusion may also be used and includes continuous subcutaneous delivery via an osmotic minipump. If so, the pharmaceutical composition may be infused at a dose between 5 and 20 µg/kg/minute, more preferably between 8 and 15 µg/kg/minute.

The following examples illustrate the invention:

### Example 1

### Generation of a modified TIF-IA mouse line

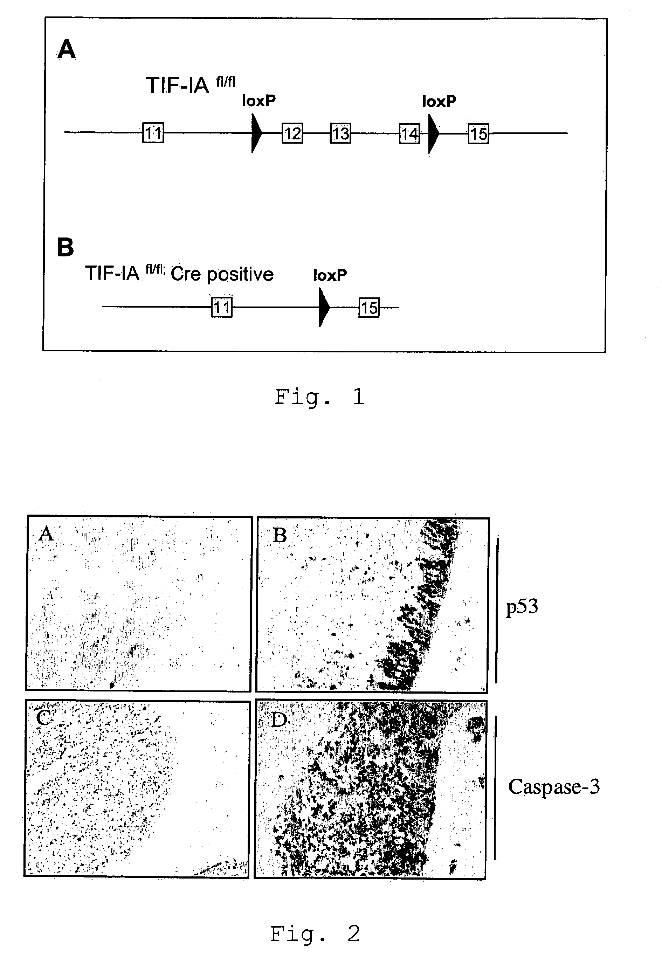

Two identical LoxP sites were introduced into the genome of the mouse in such a way that they flank exons 12 to 14 of transcription factor IA (TIF-IA) (Figure 1A). The deletion of this region is achieved by the Cre recombinase. The target vector was linearized by use of the restriction enzyme NotI and 20 µg DNA were transfected into embryonic stem cells by electroporation. Stable clones were selected and verified by Southern blotting. Two different cell lines were selected and injected into C57BI/6 blastocytes for generation of chimeric mice. By mating these heterozygote mice homozygote mice were obtained containing both alleles of the mutated TIF-IA gene (Yuan et al, Mol.Cell. 2005). Genotyping of the mice was carried out by use of PCR:
5' *Primer:* 5' - CCG GTG GTC CTG CTT ACA CTA GAG ATG TGG - 3'
*3' Primer:* 5' - AAT ATA ATT TGC AGC AGC CTG CCT GAT GAT GG - 3

### Example 2

### Ablation of TIF-IA in neural stem cells

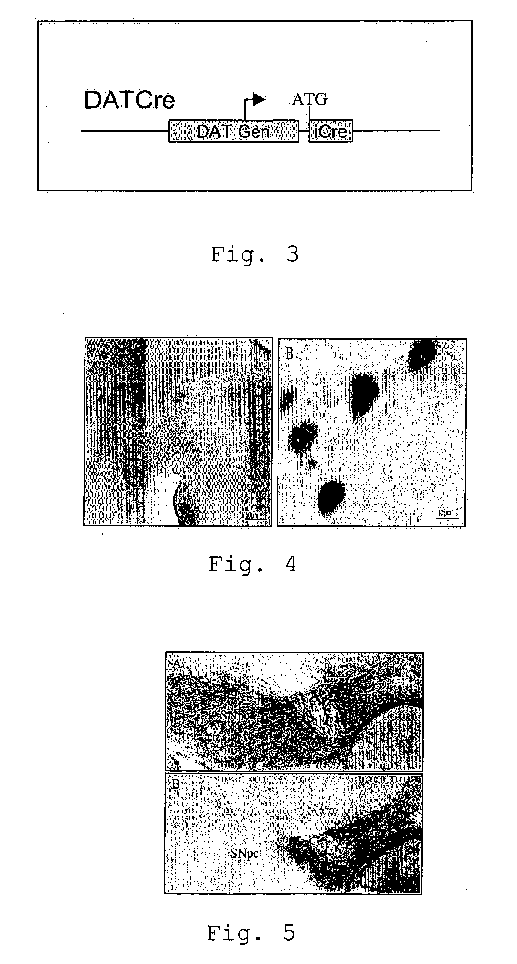

We induced the specific deletion of the TIF-IA gene in neural stem cells in the developing brain (TIF-IA^{NesCre} mutants). The NestinCre line has been previously described (Tronche et al., 1999). These mutants develop normally, but they are perinatally lethal; indeed, at birth they lack virtually all the neurons in the CNS. The deletion of the TIF-IA gene results in progressive loss of neural stem cells by activation of the apoptotic machinery mediated by p53 upregulation (Figure 2).

### Example 3

### Loss of the TIF-IA gene restricted to dopaminergic neurons

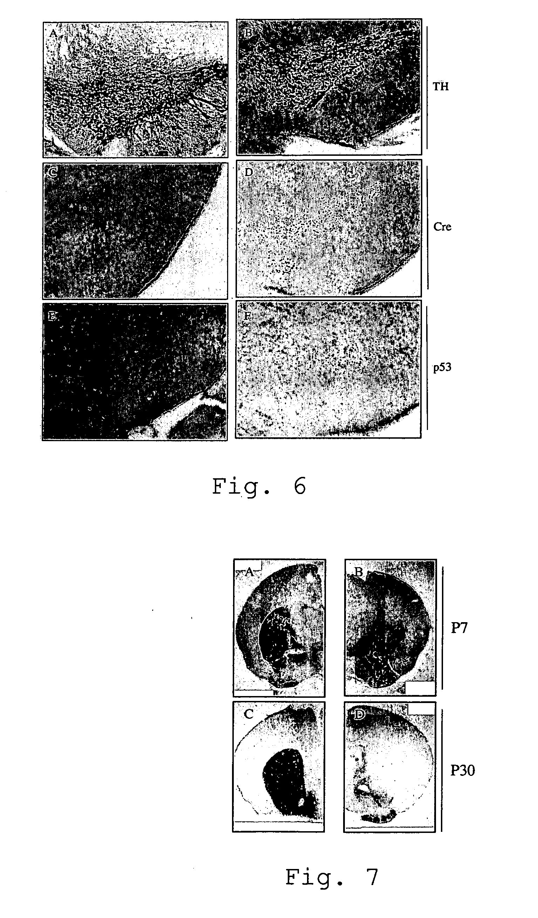

Parkinson's disease is a slowly progressing neurological disease affecting particular areas of the brain (the basal ganglions) which are involved in control of voluntary and involuntary movement. The slow degeneration of cells of the substantia nigra leads to a lack of the neurotransmitter dopamine. As a consequence, the classical symptoms of the disease are observed (main symptoms: akinesia, rigor, passive tremor and postural instability). As already mentioned earlier, so far, the experimental study of Parkinsons's disease was mainly based on model system using the neurotoxin MPTP (1-methyl-4-phenyl-1,2,3,6-tetrahydropyridine) which generates severe and irreversible symptoms in humans, primates and other mammals. However, in contrast to the MPTP based model which results in rapid apoptosis, the present mouse model shows slowly progressing degeneration of dopaminergic neurons by upregulation of the expression of the p53 protein. For getting a defined deletion of the TIF-IA gene, the TIF-IA mutants were mated with a mouse line showing tissue specific expression in dopaminergic neurons of the Cre recombinase: In this mouse line the gene encoding Cre recombinase is controlled by the dopamine transporter promoter (DAT), which is only active in dopaminergic neurons (Figures 3,4). This construct was inserted into the genome of the mouse. In the mutants obtained by mating of TIF-IA ^{fl/fl} mice and DATCre mice (TIF-IA^{fl/fl}:DATCre) expression of the Cre recombinase is restricted to the particular neurons and, thus, deletion of the TIF-IA gene is restricted to this area (Figure 1B).
PCR primer for genotyping DATCre mice
*5' Primer:* 5' - CTG CCA GGG ACA TGG CCA GG - 3'
*3' Primer:* 5' - GCA CAG TCG AGG CTG ATC AGC - 3' Brains of TIF-IA^{DATCre} mice were analyzed by immunostaining and in situ hybridization. These mice develop normally until 3 weeks after birth, thereafter this mutation results in loss of dopaminergic neurons predominantly in the substantia nigra pars compacta, as indicated by immunohistochemistry with an antibody against tyrosine-hydroxylase (TH) at P30 (Fig.5). Most likely, loss of DA neurons is due to p53-dependent apoptosis, since we have observed p53 upregulation in the dopaminergic neurons of the TIF-IA^{DATCre} mutants at P7 (Fig.6) . This process progresses until the first symptoms of Parkinson's disease can be observed (after about 5 weeks). Looking at different stages during adulthood it became clear that the mutation results in progressive loss of TH expression in the striatum starting at P7 (Fig.7). During aging of the mouse these symptoms increase. After about 8 weeks the mouse dies.

### Example 4

### Immunohistochemical analyses

Animals were sacrificed by use of CO₂, brains were taken and incubated in cold 4% Paraformaldehyde (PFA) for 48 hours. The brains were stored in 0.4% PFA at 4°C. Coronal sections (50 µm) were prepared by use of a Vibratome (Leica).

Embryos were incubated in cold 4% PFA over night and then embedded in paraffin. Sagittal sections were prepared by use of a microtome (Leica). The sections were processed for immunohistochemical detection by using the VECTASTAIN ABC system (Vector Laboratories) and diaminobenzidine (Sigma) incubation. We used the following primary antibodies: anti-Cre, 1:3000 (Mantamadiotis et al, 2002); anti-caspase-3, 1:1000 (Cell Signaling, #9661); anti-p53, 1:1000 (anti-tyrosine-hydroxylase , 1:2000 (Chemicon, AB1542). In situ hybridization was performed as previously described (Nat Neurosci. 2005 Jun;8(6):759-67).

References cited:
Bodem, J., Dobreva, G., Hoffmann-Rohrer, U., Iben, S., Zentgraf, H., Delius, H., Vingron, M., and Grummt, I. (2000). TIF-IA, the factor mediating growth-dependent control of ribosomal RNA synthesis, is the mammalian homolog of yeast Rrn3p. EMBO Rep 1, 171-175.
Hardouin, N., and Nagy, A. (2000). Gene-trap-based target site for cre-mediated transgenic insertion. Genesis 26, 245-252. Schnapp, A., Pfleiderer, C., Rosenbauer, H., and Grummt, I. (1990). A growth-dependent transcription initiation factor (TIF-IA) interacting with RNA polymerase I regulates mouse ribosomal RNA synthesis. Embo J 9, 2857-2863.
Thomson, J. A., Itskovitz-Eldor, J., Shapiro, S. S., Waknitz, M. A., Swiergiel, J. J., Marshall, V. S., and Jones, J. M. (1998). Embryonic stem cell lines derived from human blastocysts. Science 282, 1145-1147.
Tronche, F., Kellendonk, C., Kretz, O., Gass, P., Anlag, K., Orban, P. C., Bock, R., Klein, R., and Schutz, G. (1999). Disruption of the glucocorticoid receptor gene in the nervous system results in reduced anxiety. Nat Genet 23, 99-103.
Yamamoto, R. T., Nogi, Y., Dodd, J. A., and Nomura, M. (1996). RRN3 gene of Saccharomyces cerevisiae encodes an essential RNA polymerase I transcription factor which interacts with the polymerase independently of DNA template. Embo J 15, 3964-3973.

## Claims

1. Use of a non-human transgenic animal **characterized in that** it contains a modified version of the gene encoding TIF-IA (a) as an animal model for a disease, (b) for analyzing the function of selected stem cells or (c) for ablating malignant cells or microglia cells,
wherein said modified version of the gene encoding TIF-IA is a version that is cell specifically or tissue specifically modified.

2. Use according to claim 1, wherein the modified version of the gene encoding TIF-IA is a version that has been inactivated.

3. Use according to claim 1 or 2, wherein the gene encoding TIF-IA contains a deletion.

4. Use according to any one of claims 1 to 3, wherein said deletion is a deletion of exons 12 to 14.

5. Use according to claim 3 or 4, wherein said deletion has been introduced via homologous recombination in an embryonic stem cell.

6. Use according to claim 5, wherein homologous recombination is under the control of the Cre/loxP system.

7. Use according to any one of claims 1 to 6, wherein said animal is homozygous for the modified version of the gene encoding TIF-IA.

8. Use according to any one of claims 1 to 7, wherein said transgenic non-human animal is a mouse.

9. Use according to any one of claims 1 to 8, wherein said disease is Parkinson's disease, Huntington's chorea, amyotrophic lateral sclerosis, sleep-wakefulness, spinal or bulbar muscular dystrophy, diabetes or an endocrine disease.

10. A method for screening a therapeutic compound for use in the treatment of a disease comprising:
(a) administering a candidate compound to the non-human transgenic animal as defined in any one of claims 1 to 9; and
(b) monitoring a therapeutic effect of said compound.

11. A method for screening a therapeutic compound for use in the treatment of a disease comprising:
(a) administering a candidate compound to a cell line derived from the non-human transgenic animal as defined in any one of claims 1 to 9; and
(b) monitoring a therapeutic effect of said compound.
